# EUROPEAN PATENT SPECIFICATION

(11) **EP 1 281 324 B1**
(45) Date of publication and mention of the grant of the patent: **16.04.2008**
(21) Application number: 02254887.9
(22) Date of filing: 11.07.2002
(51) Int. Cl.: A23L 1/30, A23L 1/29, A23L 1/302, A23L 1/303, A23L 2/52, A23L 1/03, A23L 2/56, A61K 31/575, A61K 36/00, A61K 36/906

(54) **Health food products**
Heilnahrungsmittel
Produits alimentaires pour la santé

(30) Priority: 31.07.2001 JP 2001230909; 18.12.2001 JP 2001384860; 20.02.2002 JP 2002042639
(43) Date of publication of application: 05.02.2003
(73) Proprietor: Daicho Kikaku Incorporated Company, Shida-gun, Shizuoka 421-1132 (JP)
(72) Inventor: Ochiai, Akio, Shimizu-shi, Shizuoka 424-0926 (JP); Kosuge, Masaki, Shizuoka-shi, shizioka 420-0841 (JP); Daicho, Takao, Shida-gun, Shizuoka 421-1132 (JP)
(74) Representative: Spencer, Michael David

(56) References cited:
- EP-A- 1 198 994
- WO-A-00/59523
- WO-A-02/062367
- GB-A- 976 096
- US-A- 5 567 424
- US-A- 5 904 924
- US-B1- 6 264 995
- DATABASE WPI Section Ch, Week 199315 Derwent Publications Ltd., London, GB; Class B01, AN 1993-121276 XP002243963 & JP 05 058898 A (SUNTORY LTD), 9 March 1993 (1993-03-09)
- DATABASE WPI Section Ch, Week 199308 Derwent Publications Ltd., London, GB; Class B01, AN 1993-061599 XP002243964 & JP 05 009119 A (SUNTORY LTD), 19 January 1993 (1993-01-19)
- DATABASE WPI Section Ch, Week 199544 Derwent Publications Ltd., London, GB; Class D13, AN 1995-337328 XP002243842 & CN 1 094 592 A (FUSHAN DISTRICT INST DIETOTHERAPY YANTAI), 9 November 1994 (1994-11-09)
- DATABASE WPI Section Ch, Week 199702 Derwent Publications Ltd., London, GB; Class D13, AN 1997-018842 XP002243843 & KR 9 504 648 B (YUN Y), 4 May 1995 (1995-05-04)
- DATABASE WPI Section Ch, Week 200235 Derwent Publications Ltd., London, GB; Class D13, AN 2002-313711 XP002243844 & KR 2001 100 043 A (CHONGKUNDANGHEALTHCARE CO), 14 November 2001 (2001-11-14)
- PELUSO MICHAEL R ET AL: "A cooperative interaction between soy protein and its isoflavone-enriched fraction lowers hepatic lipids in male obese Zucker rats and reduces blood platelet sensitivity in male Sprague-Dawley rats." JOURNAL OF NUTRITION, vol. 130, no. 9, September 2000 (2000-09), pages 2333-2342, XP002243962 ISSN: 0022-3166

## Description

### TECHNICAL FIELD

The present invention relates to novel health food products. The present invention relates to novel nutritional food products useful for providing effective elements to a health conscious population.

### BACKGROUND OF THE INVENTION

Serotonin and noradrenaline referred to as brain hormones control satisfaction of the will, and in turn, spiritual satisfaction. In response to satisfaction of the will, voluntary muscles are activated by adrenaline, an adrenal medullary hormone, secreted from adrenal medulla and chromaffin cells on nerve muscles.
Serotonin, noradrenaline and adrenaline are also referred to as biogenic monoamines, and are oxidatively decomposed by an enzyme called monoamine oxidase within as long as 3 hours or reabsorbed into nerve tissues and so forth, resulting in the extinction of most effects of secreted monoamines.
This is an ingenious body mechanism that prevents fatigue produced by the long time action of monoamines.

The onset of depressive syndrome (depression) is caused by retention of a depressive condition when deprived of satisfaction of will and spirit due to a decrease in the secretory capability of biogenic monoamines, whereby the spirit is, on occasion, extremely lowered leading to strong suicidal tendencies.
Until now, for treatment of depression, passive nosotropic therapy has been carried out in which either healthy foods which inhibit an action of monoamine oxidase or healthy foods which prevent reabsorption of such monoamines are administered so that the concentration of, as a result of decrease in the secretory capability, reduced monoamines would be maintained and an extreme decrease in spirit would be prevented.
However, this therapy is not a fundamental therapy the aim of which is to eliminate the cause of depression, that is, to cure the decreased secretory ability of biogenic monoamines. Thus, it is a therapy in expectation of only naturally recovering the secretion capacity of monoamines over a long period of therapy.
Natural recovery is still a therapy with drawbacks, i.e., not only the secretory ability of monoamines is further decreased due to the continued presence of monoamines, but also physical strength is readily lowered due to fatigue caused by adrenaline-mediated excess burning of sugars and lipids.

Furthermore, "KI" (in Japanese; vital energy) refers to all the functions of the human body. Such body's functions are generically classified into 2 types. One function is controlled by autonomic nerves, which are found in various organs, hormone secretions and blood vessels. The other one works for voluntary muscles which act according to commands released from brain thought functions and thought patterns.

It has previously been pointed out that "KI", as represented by "HOKI" (in Japanese; complementing energy) and "RIKI" (in Japanese; circulating energy) in Chinese medicine, indicates only the former functions controlled by autonomic nerves. The commands for "which functions will be activated" and "how they will be exerted" are released from the medulla oblongata located at the lower part of the brain. Considering that "HOKI-YAKU" (in Japanese; drug for complementing "KI") activates all functions controlled by autonomic nerves, instead of only activating certain organs and tissues, it is likely that the target point on which the "HOKI-YAKU" acts may be medulla oblongata (a source of commands) rather than each organ or tissue. That is, it is concluded that the actions of "HOKI-YAKU" are for enhancing and facilitating the emission and transmission of commands from the medulla oblongata.

I have found that the "HOKI-YAKU" localizes body blood to various organs. This might be aimed at achieving such a blood localization via, as a result of the blood vessel-dilating and constricting action (one of the important actions of medulla oblongata), not only dilating blood vessels directed to the autonomic nervous control system but also constricting blood vessels directed to the brain and brain-controlled organs whereby more nutrient elements will be delivered to required parts as a secondary action of the "HOKI-YAKU".

In former times, depression was a rare disease. However, the onset rate of depression has recently increased worldwide, and it is said that one in 150 individuals is a depressive patient in the Tokyo Metropolitan Area.

Recently, an interesting opinion has been published concerning the causes for increases in the occurrence of depressive symptoms. Such a theory is as follows:
That is, there has been no war crisis worldwide except in some troubled regions. In addition, social order has been well maintained resulting in reducing opportunities for encountering contingent accidents.
Thus, there has been almost no need for the human body to gird itself and enhance the spirit for such dangers.
Therefore, the need for secretion of biogenic amines has also remarkably decreased, whereby the secretory capability has degenerated with the result that persons would be apt to develop depression.
If this theory is correct, when secretion of biogenic monoamines is artificially stimulated, for example using a secretomotory means once a day, the onset of depression may be prevented, the capability of secreting biogenic monoamines may be recovered even in patients whose secretion capability of biogenic monoamines is lowered, and the patient may recover from depression.
Proctoptosia, and pains in legs, lumbar regions and arms have occurred with aging. These have been considered to be due only to hematogenous disorders at the respective local sites or resultant inflammation. However, regular dosing of drug products alone which are assumed to be sufficiently capable of eliminating hematogenous disorders has been insufficient in ameliorating such conditions.
These disorders are attributed to insufficient communication of the brain commands to the local sites. Hematogenous disorders and the occurrence of inflammation are problems caused by abnormal motions of muscles due to the incomplete communication of brain commands.

From US-A-5 904 924 compositions comprising ginger, curcumin and isoflavones are known for providing energy breast.
From GB 976 096 and JP 05 058898 are known compositions comprising cholic acid, respectively scymnol sulphate for the treatment of rheumatic diseases, respectively hyperlipidemia.

### SUMMARY OF THE INVENTION

Although all muscle disorders are not ascribed to insufficient brain communication, it seems that the majority of such disorders are elicited by insufficient communication of brain commands. In particular, it has been found that muscle disorders such as backaches and pains in limbs attributable to aging are easily eliminated by curcumin and others which ameliorate the communication of brain commands.
The present inventor has succeeded in developing pharmaceutical drugs based upon such a new theory. Thus, the present invention is to provide very effective pharmaceutical drugs each exhibiting a muscle-intensifying activity and/or an anti-inflammatory activity.

The present inventor has succeeded in developing health foods based upon such a new theory. Thus, the present invention is to provide health foods which are very effective for persons who are not feeling well because of depression.

The present invention relates to health food products each comprising at least one or more members selected from a group consisting of pungent substances, bitter substances and sour substances (or acid substances), in particular to health food products each comprising at least one or more members selected from the group consisting of pungent, bitter and/or sour principles in foods.

### DETAILED DESCRIPTION OF THE INVENTION

The pungent substances as used herein is selected from curcumin 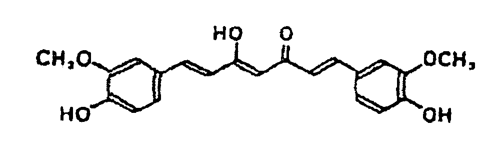 (isolated from Curcumae Rhisoma (root of *Curcuma Tonga L*.)), capsaicine 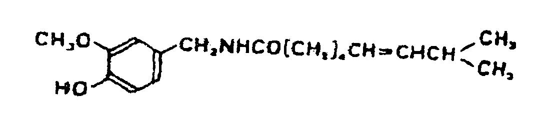 isolated from fruit of *Capsicum annuum L.*), piperine 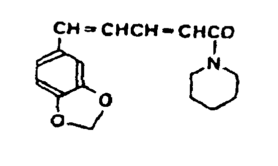 (isolated from black pepper (fruit of *Piper nigrum L*.)), zingerone 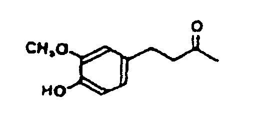 (isolated from ginger root (*Zingiber officinale Roscoe*)), (6)-shogaol 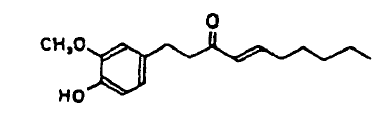 (isolated from ginger root), and (6)-gingerol 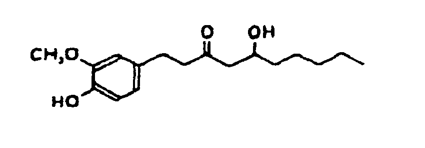 (isolated from ginger root). Among them, curcumin is most preferable.

The bitter substances as used herein include preferably swertiamarin, gentiopicrin, loganin, etc.

The sour substances as used herein include preferably citric acid, lactic acid, etc.

The daily dose of the pungent substance is preferably 1 to 1000 mg, more preferably 10 to 300 mg, and most preferably 20 to 70 mg. When it is administered alone, its daily dose is preferably 100 to 800 mg, and most preferably 300 to 500 mg. When it is administered in combination with "KAMPO" pharmaceutical preparations or drugs ("KAMPO", Japan's traditional herbal medicine) having the efficacy of " 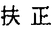 " ("FU-SEI" as pronounced in Japanese: aid for keeping the body normal), such as JUZEN-TAIHO-TO (as pronounced in Japanese), its daily dose is preferably 100 to 200 mg.

In accordance with the present invention, it is allowable that the health food product contains a pungent substance, a bitter substance, and/or a sour substance. It is preferable that the health food product is in admixture with one or more members selected from the group consisting of isoflavones, acyl isoflavones and isoflavone glycosides. The particularly preferable isoflavone includes soybean isoflavones comprised in soybean. The particularly preferable acyl isoflavone includes soybean acyl isoflavones comprised in soybean. The particularly preferable isoflavone glycoside includes soybean isoflavone glycosides comprised in soybean. For the acyl isoflavone, the preferable acyl group includes acid residues derived from saturated or unsaturated fatty acids each having 2 to 18 carbon atoms. The more preferable acyl group includes acid residues derived from acetic acid, palmitic acid, stearic acid, etc. Hydroxy groups of the isoflavone may be completely or partially acylated. Since acylation allows acylated products to have increased oil solubility, a large amount of acylated isoflavones can be dissolved in oil-based preparations.

The daily dose of isoflavone, acyl isoflavone and isoflavone glycoside is preferably 1 to 500 mg, more preferably 5 to 200 mg, and most preferably 10 to 100 mg. The daily dose of cholic acid is preferably 1 to 1000 mg, more preferably 2 to 300 mg, and most preferably 10 to 100 mg.

In accordance with the present invention, although it is allowable that the health food product contains a pungent substance, a bitter substance, and/or a sour substance, it is preferable that the health food product is in admixture with one or more members selected from the group consisting of cholic acid, scymnol and scymnol esters. Particularly, it is preferable that the health food product is in admixture with one or more members selected from the group consisting of cholic acid, scymnol and scymnol esters, in combination with one or more members selected from the group consisting of isoflavones, acyl isoflavones and isoflavone glycosides. When isoflavone, acyl isoflavone or isoflavone glycoside is administered, it is desirable that cholic acid is admixed therein.
The daily dose of cholic acid is preferably 1 to 1000 mg, more preferably 2 to 300 mg, and most preferably 10 to 100 mg. The daily dose of scymnol and scymnol esters is preferably 0.1 to 100 mg, more preferably 0.1 to 50 mg, and most preferably 0.3 to 10 mg. It is also allowable that the health food product is in admixture with other ingredients including not only generic health foods but also vitamins, heme Fe, prune extracts (*Prunus Domestica* fruit extracts), crude drugs or herbal and animal drugs (galenicals; "SHOU-YAKU" as pronounced in Japanese), and the like. The "SHOU-YAKU" includes preferably those having the efficacy of "FU-SEI", for example those capable of activating or stimulating the functions of organs, glands and blood vessels, all controlled by autonomic nerves, those capable of aiding digestion, and others.

The crude drugs of 10 or more kinds have been known as those capable of activating or stimulating the functions of organs, glands and blood vessels, all controlled by autonomic nerves. Examples of such crude drugs are ginseng (*Ginseng Radix, Panax Ginseng*), etc. Some of active elements have been revealed for not only ginseng but also such crude drugs.

Accordingly, such active elements can be preferably admixed therewith. The admixture of such active elements will lead to achievement of activating body-functions.
The particularly preferable crude drugs include Ginseng (*Panax Ginseng* or *Ginseng Radix*), *Codonopsitis Radix, Psuodostellariae Radix,* American Ginseng, *Astragali Radix*, *Atractylodis Rhizoma, Dioscoreae Rhizoma,* Glycyrrhia (*Glycyrrhizae Radix*), Jujube Fruit (*Zizyphi Fructus, Zizyphus vulgaris*), Dulcium (malt sugar derived from Oryza seed), *Polygonati Rhizoma, Codonopsis lanceolata* Benth. et Hock. *fil.* ("*SHI-YO-U-JIN*" *in Japanese; Oryza sativa* L.), etc.

Crude drugs capable of aiding digestion can be preferably admixed therewith. The particularly preferable crude drugs capable of aiding digestion include *Crataegi Fructus, Massa Medicata Fermentat, Raphani Semen, Fructus Hordei Germinatus, Fructus Oryzae Germinatus* ("KOKU-GA" in Japanese; *Oryza sativa* L.), Galli Stomachichum Corium, *Asia Foetida,* etc.

Scymnol and/or scymnol esters are comprised in biles of shark. Scymnol has the following formula:

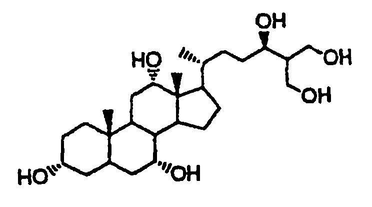

Sodium scymnol sulfate has the following formula:

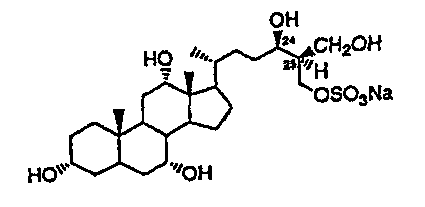

Other materials as used herein include isoflavones, acyl isoflavones and isoflavone glycosides.

For the health food products, the active elements contained in soybean are several species of isoflavone glycosides including daidzin, glycitin, genistin, etc. and aglycons thereof, i.e., several species of isoflavones including daidzein, glycitein, genistein, etc.

The soybean is a starting material for producing soybean oil. There is a great demand for soybean oil. Therefore, large amounts of soybean oil are manufactured together with large amounts of by-products, soybean cakes. Although part of such soybean cakes are employed as sources for preparing soybean proteins, etc. which are starting materials for food products, the soybean cake is mainly used for a fertilizer or feed for livestock and its price is therefore extremely low. The soybean cakes which are almost industrial wastes can be used as starting materials to produce inexpensively soybean isoflavones, soybean acyl isoflavones and soybean isoflavone glycosides with high purity.

The health food products of the present invention can be eaten as conventional forms including powdery, solid, and liquid forms. Materials for admixture include lactose, starch, vegetable oil, etc.

### EXAMPLES

Described below are examples of the present invention which are provided for illustrative purposes.
Soybean isoflavones, soybean acyl isoflavones and soybean isoflavone glycosides as used in examples are set to be 40% in purity. Cholic acid as used in examples is set to be 90% in purity except for pure cholic acid.

| EXAMPLE 1 (Powders) | |
|---|---|
| Curcumin | 45 mg |
| Scymnol | 1 mg |
| Soybean isoflavone | 125 mg |
| Lactose | 800 mg |
| Cornstarch | qs |
| Magnesium stearate | 10 mg |
| Total | 2000 mg |
| (1 g per container (powder paper), twice a day) | |

A formula except that soybean isoflavone was replaced with soybean isoflavone glycoside was blended to afford powders in the same fashion as above.

| EXAMPLE 2 (Granules) | |
|---|---|
| Curcumin | 45 mg |
| Sodium scymnol sulfate | 1 mg |
| Soybean isoflavone | 125 mg |
| Lactose | 1500 mg |
| Cornstarch | qs |
| Magnesium stearate | 10 mg |
| Total | 2000 mg |
| (1 g per container (powder paper), twice a day) | |

A formula except that soybean isoflavone was replaced with soybean isoflavone glycoside was granulated to afford granules in the same fashion as above.

| EXAMPLE 3 (Tablets) | |
|---|---|
| Curcumin | 45 mg |
| Sodium scymnol sulfate | 1 mg |
| Soybean isoflavone | 125 mg |
| Crystalline cellulose | qs |
| Lactose | 140 mg |
| Magnesium stearate | 6 mg |
| Talc | 3 mg |
| Total | 1120 mg |
| (280 mg per tablet, 2 tablets per dose, twice a day) | |

A formula except that soybean isoflavone was replaced with soybean isoflavone glycoside was compressed to afford tablets in the same fashion as above.

| EXAMPLE 4 (Tablets) | |
|---|---|
| Curcumin | 45 mg |
| Scymnol | 1 mg |
| Soybean isoflavone | 250 mg |
| Ginseng powder | 2000 mg |
| Lactose | 886 mg |
| Crystalline cellulose | qs |
| Carboxymethylcellulose calcium | 320 mg |
| Hydroxypropylcellulose | 558 mg |
| CARPLEX | 30 mg |
| Magnesium stearate | 55 mg |
| Total | 5600 mg |
| (280 mg per tablet, 5 tablets per dose, twice a day) | |

A formula except that soybean isoflavone was replaced with soybean isoflavone glycoside was compressed to afford tablets in the same fashion as above.

| EXAMPLE 5 (Hard capsules) | |
|---|---|
| Curcumin | 45 mg |
| Scymnol | 1 mg |
| Soybean isoflavone | 125 mg |
| Lactose | 218 mg |
| Cornstarch | qs |
| Magnesium stearate | 6 mg |
| Total | 1150 mg |
| (for 4 #1 capsules, 2 capsules per dose, twice a day) | |

A formula except that soybean isoflavone was replaced with soybean isoflavone glycoside was blended and packed to afford hard capsules in the same fashion as above.

| EXAMPLE 6 (Soft capsules) | |
|---|---|
| Curcumin | 45 mg |
| Sodium scymnol sulfate | 1 mg |
| Soybean isoflavone | 125 mg |
| Cod liver oil | 80 mg |
| Tocopherol acetate | 5 mg |
| Ginseng extract | 200 mg |
| Yellow beeswax | 55 mg |
| Edible oil | qs |
| Total | 1200 mg |
| (4 capsules a day) | |

A formula except that soybean isoflavone was replaced with acetylated soybean isoflavone or soybean isoflavone glycoside was blended and packed to afford soft capsules in the same fashion as above.

| EXAMPLE 7 (Drink) | |
|---|---|
| Curcumin | 45 mg |
| Sodium scymnol sulfate | 1 mg |
| Soybean isoflavone | 125 mg |
| Korean ginseng extract | 10 mg |
| Rehmanniae Radix extract | 10 mg |
| (*Rehmannia glutinosa Liboschitz var. purpurea* Makino) | |
| Royal jelly | 100 mg |
| Thiamin nitrate | 10 mg |
| Riboflavin sodium phosphate | 5 mg |
| Pyridoxine hydrochloride | 10 mg |
| Anhydrous caffeine | 50 mg |
| Ethanol | 1.2 mL |
| Ethyl parahydroxybenzoate | 4 mg |
| Purified water | qs |
| Total | 50 mL/bottle |

A formula except that soybean isoflavone was replaced with soybean isoflavone glycoside was mixed to afford drinks in the same fashion as above.

| EXAMPLE 8 (Preparations admixed with vitamin, hard capsules) | |
|---|---|
| Curcumin | 45 mg |
| Sodium scymnol sulfate | 1 mg |
| Soybean isoflavone | 125 mg |
| Thiamin hydrochloride | 25 mg |
| Pyridoxine hydrochloride | 10 mg |
| Riboflavin | 10 mg |
| Cyanocobalamin | 12 µg |
| Nicotinamide | 20 mg |
| Calcium pantothenate | 20 mg |
| Ascorbic acid | 60 mg |
| L-cysteine | 10 mg |
| Lactose | 263 mg |
| Magnesium stearate | 6 mg |
| Cornstarch | qs |
| Total | 1150 mg |
| (2 capsules per dose, twice a day) | |

A formula except that soybean isoflavone was replaced with soybean isoflavone glycoside was blended and packed to afford hard capsules in the same fashion as above.

| EXAMPLE 9 (Powders) | |
|---|---|
| Curcumin | 45 mg |
| Cholic acid | 60 mg |
| Soybean isoflavone glycoside | 125 mg |
| Lactose | 2700 mg |
| Cornstarch | qs |
| Light anhydrous silicic acid | 5 mg |
| Magnesium stearate | 10 mg |
| Total | 4000 mg |
| (2 g per container (powder paper), twice a day) | |

A formula except that soybean isoflavone glycoside was replaced with soybean isoflavone was blended to afford powders in the same fashion as above.

| EXAMPLE 10 (Granules) | |
|---|---|
| Curcumin | 45 mg |
| Cholic acid | 60 mg |
| Soybean isoflavone glycoside | 125 mg |
| Lactose | 2700 mg |
| Cornstarch | 800 mg |
| Crystalline cellulose | 300 mg |
| Light anhydrous silicic acid | 5 mg |
| Magnesium stearate | 10 mg |
| Total | 4000 mg |

A formula except that soybean isoflavone glycoside was replaced with soybean isoflavone was granulated to afford granules in the same fashion as above.

| EXAMPLE 11 (Spherical granules) | |
|---|---|
| Curcumin | 45 mg |
| Cholic acid | 60 mg |
| Soybean isoflavone glycoside | 125 mg |
| Lactose | 515 mg |
| Cornstarch | qs |
| "KAN-BAI-KO" (in Japanese) | 500 mg |
| (*Prunus mume* fruit powder) | |
| Crystalline cellulose | 400 mg |
| Total | 2000 mg |

A formula except that soybean isoflavone glycoside was replaced with soybean isoflavone was granulated to afford spherical granules in the same fashion as above.

| EXAMPLE 12 (Tablets) | |
|---|---|
| Curcumin | 45 mg |
| Cholic acid | 140 mg |
| Soybean isoflavone glycoside | 280 mg |
| Lactose | 4000 mg |
| Carboxymethylcellulose calcium | 320 mg |
| Hydroxypropylcellulose | 74 mg |
| Crystalline cellulose | qs |
| CARPLEX | 30 mg |
| Magnesium stearate | 10 mg |
| Total | 5484 mg |
| (280 mg per tablet, 5 tablets per dose, twice a day) | |

A formula except that soybean isoflavone glycoside was replaced with soybean isoflavone was compressed to afford tablets in the same fashion as above.

| EXAMPLE 13 (Hard capsules) | |
|---|---|
| Curcumin | 45 mg |
| Cholic acid | 60 mg |
| Soybean isoflavone glycoside | 125 mg |
| Cornstarch | qs |
| Magnesium stearate | 9 mg |
| Total | 1153 mg |
| (#1 capsule, 4 capsules a day) | |

A formula except that soybean isoflavone glycoside was replaced with soybean isoflavone was blended and packed to afford hard capsules in the same fashion as above.

| EXAMPLE 14 (Soft capsules) | |
|---|---|
| Curcumin | 45 mg |
| Cholic acid | 60 mg |
| Soybean isoflavone glycoside | 125 mg |
| Yellow beeswax | 55 mg |
| Edible oil | qs |
| Total | 1200 mg |
| (4 capsules for a daily dose) | |

A formula except that soybean isoflavone glycoside was replaced with acetylated soybean isoflavone or soybean isoflavone was blended and packed to afford soft capsules in the same fashion as above.

| EXAMPLE 15 (Drink) | |
|---|---|
| Curcumin | 45 mg |
| Cholic acid | 60 mg |
| Soybean isoflavone glycoside | 125 mg |
| Korean ginseng extract | 1500 mg |
| *Euphoria longan* extract | 100 mg |
| Schizandrae Fructus fluidextract | 300 mg |
| (fruit of *Schizandra chinensis* Baill.) | |
| Royal jelly | 150 mg |
| Riboflavin sodium phosphate | 10 mg |
| Ethanol | 1.2 ml |
| Parahydroxybenzoic acid | 4 mg |
| Purified water | qs |
| Total | 50 ml |

A formula except that soybean isoflavone glycoside was replaced with soybean isoflavone was mixed to afford drinks in the same fashion as above.

| EXAMPLE 16 (Granules) | |
|---|---|
| Curcumin | 45 mg |
| Cholic acid | 60 mg |
| Soybean isoflavone glycoside | 125 mg |
| Thiamin hydrochloride | 10 mg |
| Pyridoxine hydrochloride | 100 mg |
| Hydroxocobalamin hydrochloride | 1.027 mg |
| Tocopherol acetate | 100 mg |
| Lactose | 2700 mg |
| Crystalline cellulose | 300 mg |
| Light anhydrous silicic acid | 5 mg |
| Magnesium stearate | 10 mg |
| Cornstarch | qs |
| Total | 4000 mg |
| (2 g per container (powder paper), twice a day) | |

A formula except that soybean isoflavone glycoside was replaced with soybean isoflavone was granulated to afford granules in the same fashion as above.

| EXAMPLE 17 (Capsules) | |
|---|---|
| Curcumin | 45 mg |
| Cholic acid | 60 mg |
| Soybean isoflavone glycoside | 125 mg |
| Vitamin A oil | 4 mg |
| Cholecalciferol | 0.005 mg |
| Tocopherol acetate | 10 mg |
| Vitamin C | 600 mg |
| Crystalline cellulose | 250 mg |
| Magnesium stearate | 6 mg |
| Cornstarch | qs |
| Total | 1150 mg |
| (#1 capsule, 4 capsules a day). | |

A formula except that soybean isoflavone glycoside was replaced with soybean isoflavone was blended and packed to afford capsules in the same fashion as above.

| EXAMPLE 18 (Powders) | |
|---|---|
| Curcumin | 30 mg |
| Scymnol | 1 mg |
| Soybean isoflavone | 125 mg |
| Lactose | 800 mg |
| Cornstarch | qs |
| Magnesium stearate | 10 mg |
| Total | 2000 mg |

A formula except that soybean isoflavone was replaced with soybean isoflavone glycoside was blended to afford powders in the same fashion as above.

| EXAMPLE 19 (Granules) | |
|---|---|
| Curcumin | 30 mg |
| Sodium scymnol sulfate | 1 mg |
| Soybean isoflavone | 125 mg |
| Lactose | 1500 mg |
| Cornstarch | qs |
| Magnesium stearate | 10 mg |
| Total | 2000 mg |

A formula except that soybean isoflavone was replaced with soybean isoflavone glycoside was granulated to afford granules in the same fashion as above.

| EXAMPLE 20 (Tablets) | |
|---|---|
| Curcumin | 25 mg |
| Scymnol | 1 mg |
| Soybean isoflavone | 250 mg |
| Ginseng powder | 2000 mg |
| Lactose | 886 mg |
| Crystalline cellulose | qs |
| Carboxymethylcellulose calcium | 320 mg |
| Hydroxypropylcellulose | 558 mg |
| CARPLEX | 30 mg |
| Magnesium stearate | 55 mg |
| Total | 5600 mg |

A formula except that soybean isoflavone was replaced with soybean isoflavone glycoside was compressed to afford tablets in the same fashion as above.

| EXAMPLE 21 (Soft capsules) | |
|---|---|
| Curcumin | 25 mg |
| Sodium scymnol sulfate | 1 mg |
| Soybean isoflavone | 125 mg |
| Cod liver oil | 80 mg |
| Tocopherol acetate | 5 mg |
| Ginseng extract | 200 mg |
| Yellow beeswax | 55 mg |
| Edible oil | gs |
| Total | 1200 mg |

A formula except that soybean isoflavone was replaced with acetylated soybean isoflavone or soybean isoflavone glycoside was blended and packed to afford soft capsules in the same fashion as above.

| EXAMPLE 22 (Drink) | |
|---|---|
| Curcumin | 30 mg |
| Sodium scymnol sulfate | 1 mg |
| Soybean isoflavone | 125 mg |
| Korean ginseng extract | 10 mg |
| Rehmanniae Radix extract | 10 mg |
| Royal jelly | 100 mg |
| Thiamin nitrate | 10 mg |
| Riboflavin sodium phosphate | 5 mg |
| Pyridoxine hydrochloride | 10 mg |
| Anhydrous caffeine | 50 mg |
| Ethanol | 1.2 L |
| Ethyl parahydroxybenzoate | 4 mg |
| Purified water | qs |
| Total | 50 mL/bottle |

A formula except that soybean isoflavone was replaced with soybean isoflavone glycoside was mixed to afford drinks in the same fashion as above.

| EXAMPLE 23 (Powders) | |
|---|---|
| Curcumin | 30 mg |
| Cholic acid | 60 mg |
| Soybean isoflavone glycoside | 125 mg |
| Lactose | 2700 mg |
| Cornstarch | qs |
| Light anhydrous silicic acid | 5 mg |
| Magnesium stearate | 10 mg |
| Total | 4000 mg |
| (2 g per powder paper, twice a day) | |

A formula except that soybean isoflavone glycoside was replaced with soybean isoflavone was blended to afford powders in the same fashion as above.

| EXAMPLE 24 (Granules) | |
|---|---|
| Curcumin | 25 mg |
| Cholic acid | 60 mg |
| Soybean isoflavone glycoside | 125 mg |
| Lactose | 2700 mg |
| Cornstarch | qs |
| Crystalline cellulose | 300 mg |
| Light anhydrous silicic acid | 5 mg |
| Magnesium stearate | 10 mg |
| Total | 4000 mg |

A formula except that soybean isoflavone glycoside was replaced with soybean isoflavone was granulated to afford granules in the same fashion as above.

| EXAMPLE 25 (Tablets) | |
|---|---|
| Curcumin | 30 mg |
| Cholic acid | 140 mg |
| Soybean isoflavone glycoside | 280 mg |
| Lactose | 4000 mg |
| Carboxymethylcellulose calcium | 320 mg |
| Hydroxypropylcellulose | 74 mg |
| Crystalline cellulose | qs |
| CARPLEX | 30 mg |
| Magnesium stearate | 10 mg |
| Total | 5484 mg |
| (280 mg per tablet, 5 tablets per dose, twice a day) | |

A formula except that soybean isoflavone glycoside was replaced with soybean isoflavone was compressed to afford tablets in the same fashion as above.

| EXAMPLE 26 (Hard capsules) | |
|---|---|
| Curcumin | 25 mg |
| Cholic acid | 60 mg |
| Soybean isoflavone glycoside | 125 mg |
| Cornstarch | qs |
| Magnesium stearate | 9 mg |
| Total | 1153 mg |
| (4 #1 capsules a day) | |

A formula except that soybean isoflavone glycoside was replaced with soybean isoflavone was blended and packed to afford hard capsules in the same fashion as above.

| EXAMPLE 27 (Drink) | |
|---|---|
| Curcumin | 30 mg |
| Cholic acid | 60 mg |
| Soybean isoflavone glycoside | 125 mg |
| Korean ginseng extract | 1500 mg |
| *Euphoria longan* extract | 100 mg |
| Schizandrae Fructus fluidextract | 300 mg |
| Royal jelly | 150 mg |
| Riboflavin sodium phosphate | 10 mg |
| Ethanol | 1.2 ml |
| Parahydroxybenzoic acid | 4 mg |
| Purified water | qs |
| Total | 50 ml |

A formula except that soybean isoflavone glycoside was replaced with soybean isoflavone was mixed to afford drinks in the same fashion as above.

No studies have been conducted on the efficacy of curcumin, zingerone, or shogaol on the stimulation of adrenaline secretion. However, the secretomotory actions on adrenaline have been clarified through animal experiments using mice in which blood glucose levels were remarkably elevated.
Tablets (250 mg each) containing curcumin (15 mg), cholic acid (20 mg) and soybean isoflavone glycoside (40 mg) in admixture with lactose were administered at a dose of 3 tablets a day (at a dose of 45 mg of curcumin once a day) to 10 patients who had regularly received drugs having the efficacy of "FU-SEI" (in Japanese). The results are shown in the following Table:

**Table 1**

| | Age | Sex | Pathological State | Drug Regularly Dosed | | | Hospitalization | Efficacy |
|---|---|---|---|---|---|---|---|---|
| | | | | Antidepressant | Tranquilizer | Hypnotic | | |
| 1 | 51 | Male | Severe Depression, Anxiety | ○ | ○ | ○ | ○ | ⊚ |
| 2 | 38 | Male | Insomnia, Anxiety, Irritableness | ○ | ○ | ○ | ○ | ○ |
| 3 | 51 | Male | Insomnia, Anxiety | × | × | × | × | - |
| 4 | 30 | Female | Insomnia, Anxiety, Depression | ○ | ○ | ○ | × | ⊚ |
| 5 | 18 | Female | Insomnia, Anxiety, Irritableness | × | × | × | × | ⊚ |
| 6 | 76 | Male | Irritableness | × | × | × | × | ○ |
| 7 | 74 | Female | Intensive Anxiety, Insomnia, Irritableness | × | × | × | × | ○ |
| 8 | 48 | Female | Depression, Excessive sleeping | ○ | ○ | × | × | ⊚ |
| 9 | 48 | Male | Psychomotor detardation, Irritableness, Intensive Depression | × | × | × | × | ⊚ |
| 10 | 39 | Female | Intensive Depression, Insomnia, Anxiety | × | × | × | × | ⊚ |

| | | | | | | | | |
|---|---|---|---|---|---|---|---|---|
| 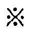 All patients were diagnosed as suffering from depression by specialized physicians. 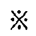 For efficacy: ○, effective; ⊚, significantly effective; - , follow-up | | | | | | | | |

As shown in the above Table, the drug is effective in 90% of the cases and the significantly effective case equals 60%.
These good results indicate the possibility of recovery from depression which had been said to be a non-curable disease. The examples as disclosed in the Table show results obtained from dosage trials over two months after the initiation of administration; however, long term observation is required for recurrence of depression. Thus, it has been proven that many curcumin-dosed patients are able to return to daily life routines nearly identical to those of ordinary people (non-sufferers of depression), demonstrating that these healthy foods are extremely effective and invigorating.
When tablets (250 mg each) containing, in admixture with lactose, sodium scymnol sulfate (1 mg) in place of cholic acid were administered, similar results were obtained.

Granules of Examples 2 and 10 were administered to 12 and 14 females with menopausal disorders including broodiness, depression and dizziness, etc., respectively, at 45 mg of curcumin per day. Five days after the dosing, the broodiness, depression and dizziness were mitigated in five and six individuals, respectively. Three weeks later, improvements were seen in 11 and 12 individuals, respectively.
When granules of Example 2 were administered to 10 elderly patients in the preliminary stages of senile dementia, memory loss was ameliorated in 7 individuals. When granules of Example 10 were administered, similar results were obtained. Such products are also effective for Alzheimer's disease.
It has been clarified that the products according to the present invention are significantly useful as prophylactic or therapeutic drugs for climacteric disorders, senile dementia and Alzheimer's disease. Thus, methods are provided according to the present invention, for preventing or treating a member selected from the group consisting of climacteric disorders, senile dementia and Alzheimer's disease.
Curcumin is readily available. For instance, highly pure curcumin is on the market.
Capsules wherein powders produced by admixture of capsaicine (50 mg) with lactose were packed in combination with isoflavone glycoside and cholic acid were administered to patients with depression once daily. Several days later, effects were dramatic. The patient were nearly completely recovered from depression after a week. Capsaicine is a powerful irritant with burning aftertaste.

Tablets of Example 20 were administered once a day.
For an eighty-three year old male afflicted with walking difficulties and severe anal prolapse, occurred as he was aging, the dosing led to easy mobility and recovery from proctoptosia, with a clear feeling that the anal sphincter functioned. One-month dosing led to clear amelioration of his conditions.
For a male (age 63) afflicted with difficulty in walking up and down stairs, the dosing led to easy movement on staircases. One-month dose trials led to clear amelioration. The tablet of example 25 has a similar efficacy.

If these results were simply attributable to pharmacological action to improve local muscle activity, such information would be contained in European and American data and in results found in Okinawa, wherein curcumin is applied. No such data exists therein. This indicates that these results might be attributed to improvements in brain communication of commands (released as a result of brain thought) to local sites.
In Western medicine, it has been understood that proctoptosia and leg, lumbus and arm aches are attributable only to hematogenous disorders and resultant inflammation at local sites. However, these pathological conditions were not significantly ameliorated by a single regular dose of drugs admixed with one of scymnol, isoflavone, and cholic acid which are significantly capable of removing hematogenous disorders. Thus, it can be understood that these disorders are attributed to insufficient communication of brain commands to local sites and therefore the hematogenous disorders and onset of inflammation are problems caused by abnormal motions of muscles due to incomplete communication of brain commands.

When male and female patients (5 each, age 60 to 75) complained of backaches, limb pains and muscle disorders such as proctoptosia (which occurred as they were aging) took doses, such clinical conditions were alleviated or cured.
Although all muscle disorders are not ascribable to insufficient brain communication, it seems that the majority of such disorders are elicited by insufficient communication of brain commands.

Coadministration of isoflavone, cholic acid, scymnol, curcumin and so on enables the removal of muscle disorders as such components have the stimulating actions on brain activity. This is good news to the elders for both physical and mental rejuvenation. These formulations are extremely effective for arthritis such as rheumatism and in particular, greatly influence the prevention.
Curcumin is readily available. For instance, highly pure curcumin is on the market.

Capsaicine (30 mg) instead of curcumin preparations of Example 18 was administered to elderly patients between the ages of 76 and 83 once daily. Several days later, its effects were dramatic. It has been proved that the drug is effective for muscular degeneration, arthritis and rheumatism after one week.

### ADVANTAGES OF THE PRESENT INVENTION

The present invention is to provide very effective health foods for supplying a pungent substance, a bitter substance or a sour substance, thereby refreshing a body or feeling, preventing forgetting, and intensifying weakened muscles. Thus the health food products are advantageous in view of prevention or treatment of rheumatism, etc.

## Claims

1. A health food product comprising at least one pungent substance, one or more members selected from the group consisting of cholic acid, scymnol and scymolesters, and one or more members selected from the group consisting of isoflavones, acyl isoflavones and isoflavone glycosides.

2. A health food product according to claim 1, **characterised in that** the pungent substance is selected from foods.

3. A health food product according to claim 1 or claim 2, **characterised in that** the pungent substance is curcumin.

4. A health food product according to any preceding claim, **characterised in that** the isoflavone is a soybean isoflavone, the acyl isoflavone is a soybean acyl isoflavone and the isoflavone glycoside is a soybean isoflavone glycoside.

5. A health food product according to any preceding claim which is in admixture with a vitamin.

6. A health food product according to any preceding claim which is in admixture with at least one or more members selected from crude drugs.

7. A health food product according to claim 6, **characterised in that** the crude drug is one or more members selected from the group consisting of Ginseng, *Codonopsitis Radix, Psuodostellariae Radix,* American Ginseng, *Astragali Radix, Atractylodis Rhizoma, Dioscoreae Rhizoma,* Glycyrrhia, Jujube Fruit, *Dulcium, Polygonati Rhizoma,* and *Codonopsis lanceolata* Benth. et Hock. *fil.* .

8. A health food product according to claim 6 or claim 7, **characterised in that** the crude drug is one or more members selected from the group consisting of *Cratagei Fructus*, *Massa Medicata Fermentat, Raphani Semen, Fructus Hordei Germinatus, Fructus Oryzae Germinatus* known as "KOKU-GA" in Japanese or *Oryza sativa* L.), Galli Stomachichum Corium, and *Asa Foetida.*

9. A health food product according to claim 1, **characterised in that** the pungent substance is selected from the group consisting of curcumin, capsaicine, piperine, zingerone, (6)-shogaol, and (6)-gingerol.

10. A health food product according to claim 9, **characterised in that** the pungent substance is curcumin.

## Patentansprüche

1. Gesundheitsförderndes Nahrungsmittel, Heilnahrungsmittel oder Nahrungsergänzungsmittel, umfassend mindestens eine scharf schmeckende Substanz, eine oder mehrere Substanzen, ausgewählt aus der aus Cholsäure, Scymnol und Scymnolestern bestehenden Gruppe, und eine oder mehrere Substanzen, ausgewählt aus der aus Isoflavonen, Acylisoflavonen und Isoflavon-Glycosiden bestehenden Gruppe.

2. Gesundheitsförderndes Nahrungsmittel, Heilnahrungsmittel oder Nahrungsergänzungsmittel gemäß Anspruch 1, **dadurch gekennzeichnet, dass** die scharf schmeckende Substanz unter Lebensmitteln ausgewählt ist.

3. Gesundheitsförderndes Nahrungsmittel, Heilnahrungsmittel oder Nahrungsergänzungsmittel gemäß Anspruch 1 oder Anspruch 2, **dadurch gekennzeichnet, dass** die scharf schmeckende Substanz Curcumin ist.

4. Gesundheitsförderndes Nahrungsmittel, Heilnahrungsmittel oder Nahrungsergänzungsmittel nach einem der voranstehenden Ansprüche, **dadurch gekennzeichnet, dass** das Isoflavon ein Sojabohnen-Isoflavon ist, dass das Isoflavon ein Sojabohnen-Acylisoflavon ist und das Isoflavon-Glycosid ein Sojabohnen-Isoflavon-Glycosid ist.

5. Gesundheitsförderndes Nahrungsmittel, Heilnahrungsmittel oder Nahrungsergänzungsmittel nach einem der voranstehenden Ansprüche, das in Mischung mit einem Vitamin vorliegt.

6. Gesundheitsförderndes Nahrungsmittel, Heilnahrungsmittel oder Nahrungsergänzungsmittel nach einem der voranstehenden Ansprüche, das sich in Mischung mit mindestens einem oder mehreren Substanzen befindet, die unter unbearbeiteten oder ungekochten Drogen bzw. Arzneimitteln ausgewählt sind.

7. Gesundheitsförderndes Nahrungsmittel, Heilnahrungsmittel oder Nahrungsergänzungsmittel gemäß Anspruch 6, **dadurch gekennzeichnet, dass** das unbearbeitete oder ungekochte Arzneimittel bzw. diese Droge eine oder mehrere Substanzen ist, ausgewählt aus der aus Ginseng, *Codonopsitis Radix, Psuodostellariae Radix,* American Ginseng, *Astragali Radix, Atractylodis Rhizoma, Dioscoreae Rhizoma, Glycyrrhia,* der Frucht der Indischen Brustbeere, *Dulcium, Polygonati Rhizoma* und *Codonopsis Lanceolata* Benth. et Hock. *fil.* bestehenden Gruppe.

8. Gesundheitsförderndes Nahrungsmittel, Heilnahrungsmittel oder Nahrungsergänzungsmittel gemäß Anspruch 6 oder Anspruch 7, **dadurch gekennzeichnet, dass** das unbearbeitete oder ungekochte Arzneimittel bzw. diese Droge eine oder mehrere Substanzen ist, ausgewählt aus der aus *Cratagei Fructus, Massa Medicata Fermentat, Raphani Semen, Fructus Hordei Germinatus, Fructus Oryzae Germinatus* (bekannt als "KOKU-GA" auf Japanisch oder Oryza Sativa L.), *Galli Stomachichum Corium* und *Asa Foetida* bestehenden Gruppe.

9. Gesundheitsförderndes Nahrungsmittel, Heilnahrungsmittel oder Nahrungsergänzungsmittel gemäß Anspruch 1, **dadurch gekennzeichnet, dass** die scharf schmeckende Substanz ausgewählt ist aus der aus Curcumin, Capsaicin, Piperin, Zingeron, (6)-Shogaol und (6)-Gingerol bestehenden Gruppe.

10. Gesundheitsförderndes Nahrungsmittel, Heilnahrungsmittel oder Nahrungsergänzungsmittel gemäß Anspruch 9, **dadurch gekennzeichnet, dass** die scharf schmeckende Substanz Curcumin ist.

## Revendications

1. Produit alimentaire pour la santé comprenant au moins une substance âcre, un ou plusieurs membres choisis dans le groupe consistant en l'acide cholique, le scymnol et les esters du scymnol, et un ou plusieurs membres choisis dans le groupe consistant en les isoflavones, les acylisoflavones et les glycosides d'isoflavone.

2. Produit alimentaire pour la santé selon la revendication 1, **caractérisé en ce que** la substance âcre est choisie parmi les produits alimentaires.

3. Produit alimentaire pour la santé selon la revendication 1 ou 2, **caractérisé en ce que** la substance âcre est le curcumin.

4. Produit alimentaire pour la santé selon l'une quelconque des revendications précédentes, **caractérisé en ce que** l'isoflavone est une isoflavone du soja, l'acylisoflavone est une acylisoflavone du soja, et le glycoside d'isoflavone est un glycoside d'isoflavone du soja.

5. Produit alimentaire pour la santé selon l'une quelconque des revendications précédentes, qui est en mélange avec une vitamine.

6. Produit alimentaire pour la santé selon l'une quelconque des revendications précédentes, qui est en mélange avec au moins un ou plusieurs membres choisis parmi les médicaments bruts.

7. Produit alimentaire pour la santé selon la revendication 6, **caractérisé en ce que** le médicament brut est un ou plusieurs membres choisis dans le groupe consistant en le ginseng, *Codonopsitis Radix, Psuodostellariae Radix,* le ginseng américain, *Astragali Radix, Atractylodis Rhizoma, Dioscoreae Rhizoma*, glycyrrhia, le fruit de jujube, *Dulcium, Polygonati Rhizoma* et *Codonopsis lanceolata* Benth. et Hock. *fil*.

8. Produit alimentaire pour la santé selon la revendication 6 ou 7, **caractérisé en ce que** le médicament brut est un ou plusieurs membres choisis dans le groupe consistant en *Cratagei Fructus, Massa Medicata Fermentat, Raphani Semen*, *Fructus Hordei Germinatus, Fructus Oryzae Germinatus,* connu sous le nom de "KOKU-GA" au Japon, ou *Oryza sativa L.*, *Galli Stomachichum Corium* et *Asa Foetida.*

9. Produit alimentaire pour la santé selon la revendication 1, **caractérisé en ce que** la substance âcre est choisie dans le groupe consistant en le curcumin, la capsaïcine, la pipérine, la zingérone, le (6)-shogaol et le (6)-gingérol.

10. Produit alimentaire pour la santé selon la revendication 9, **caractérisé en ce que** la substance âcre est le curcumin.
